(19) Europäisches Patentamt / European Patent Office / Office européen des brevets

(11) **EP 2 696 191 A1**

(12) **EUROPEAN PATENT APPLICATION**
published in accordance with Art. 153(4) EPC

(43) Date of publication:
**12.02.2014 Bulletin 2014/07**

(51) Int Cl.:
**G01N 21/00** (2006.01)    **G06T 1/40** (2006.01)
**G01J 3/40** (2006.01)    **G06F 19/00** (2011.01)
**A61B 6/00** (2006.01)

(21) Application number: **12767459.6**

(22) Date of filing: **10.04.2012**

(86) International application number:
**PCT/ES2012/070239**

(87) International publication number:
**WO 2012/136874 (11.10.2012 Gazette 2012/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.04.2011 ES 201130548 P**

(71) Applicants:
• **Universitat Autònoma De Barcelona**
 **08193 Barcelona (ES)**
• **Masaryk University**
 **601 77 Brno (CZ)**
• **Università La Sapienza**
 **00185 Roma (IT)**

(72) Inventors:
• **BLANCO, Francisco**
 **E-08193 Cerdanyola del Vallès (ES)**
• **BONIFAZI, Giuseppe**
 **I-00185 Roma (IT)**
• **GARGIULO, Aldo**
 **I-00185 Roma (IT)**
• **HAVEI, Josef**
 **616 00 Brno (CZ)**
• **LÓPEZ, Montserrat**
 **E-08193 Cerdanyola del Vallès (ES)**
• **SERRANTI, Silvia**
 **I-00185 Roma (IT)**
• **VALIENTE, Manuel**
 **E-08193 Cerdanyola del Vallès (ES)**

(54) **METHOD FOR THE CHARACTERISATION AND CLASSIFICATION OF KIDNEY STONES**

(57)    Method for the characterisation and classification of kidney stones, comprised the following stages:

(a) Taking a series of kidney stone samples and cutting them to observe their interior, obtaining the flattest possible surface,

(b) The technique of Hyperspectral Imaging (HSI) is applied to obtain the spectra of previously cut kidney stones, selecting a series of Regions of Interest (ROI) and analysing the image using Principal Component Analysis (PCA)

(c) The main species are identified using Factor Analysis (FA)

(d) Outliers are identified using Principal Component Analysis (PCA)

(e) The different types of kidney stones are analysed using Principal Component Analysis (PCA)

(f) The data obtained from the Principal Component Analysis (PCA) are subject to Artificial Neural Networks (ANN) for classification.

## Description

### Object of the Invention

This invention relates to a method for the characterisation and classification of kidney stones through analysis using Hyperspectral Imaging (HSI) and Artificial Neural Networks (ANN), thus enabling high measurement speeds, and not requiring complex previous preparation of the initial samples.

### Background of the invention

The formation of kidney stones is a disorder that affects a high percentage of the population. A large range of substances can be found as the main components of kidney stones; therefore their characterisation is of enormous importance in finding the causes of this problem. In this regard, analysis of the composition and structure of the stone may provide key information about the urine conditions during crystal growth.

Many studies have been carried out in this field, basically aimed at reducing the incidence rate and increasing the quality of life of patients suffering the disorder while at the same time cutting the medical costs for related treatment and surgery. A careful study of the structure of an expelled kidney stone can provide key information about the process of its formation and the urine conditions during its growth. Therefore, by specifically analysing the nucleus and outer shell of the sample, it is possible to determine the compound acting as the precipitation core and the substances that precipitate at a later stage.

Many methods have been developed for classifying kidney stones. The most widespread methods are the examination of kidney stones by stereo microscopy and IR analysis. The former has the major advantage of allowing identification of different substances in the whole area of the sample, so it is possible to determine all the morphological characteristics of the stone. However, this method is complex and, more significantly, highly dependent on the skills of the operator. The IR methods developed to date require grinding the sample, and this process means that any spatial analysis loses all meaning.

Furthermore, some studies carried out to date on kidney stone structure include other techniques such as NIR Spectroscopy, Scanning Electron Microscope - Energy Dispersive X ray Spectroscopy (SEM-EDS) and even X-ray diffraction. NIR spectroscopy shows good performance for determining the composition of kidney stones, and even the quantitation of mixtures. Nevertheless, these studies still require a certain pre-treatment of the sample, making this type of analysis impractical in a hospital setting.

### Disclosure of the invention

The invention described here is a new method for the characterisation and classification of kidney stones based on Hyperspectral Imaging to study the kidney stones, in combination with Artificial Neural Network (ANN) analysis. The NIR reflectance spectra of stones analysed show significant differences that can be used for their classification. The possibility of using Artificial Neural Networks for classifying kidney stones was also assessed. The results show more than 90% of correct classification for all types of stones, including those that are mixtures of different compounds. These promising results show the good performance of the method developed and provide new prospects to improve the patients' quality of life.

The kidney stone characterisation procedure described here uses the technique of Hyperspectral or Chemical Imaging (HSI) that combines a camera with a spectrometer to enable capturing a spectrum for each pixel of the acquired image, thus making it possible to characterise the entire surface of the sample. Regarding the importance of spatial characterisation of the sample, Hyperspectral or Chemical Imaging (HSI) is extremely useful for this purpose as it enables characterisation of really small areas on the sample surface. Apart from spatial resolution, hyperspectral imaging enables high measurement rates and very little previous preparation of the sample. Essentially, Hyperspectral or Chemical Imaging (HSI) is an interesting possibility for the analysis of kidney stone samples in a hospital setting.

The management of the enormous amounts of raw data available from Hyperspectral Imaging requires some chemometric techniques. Principal Component Analysis (PCA) is commonly used to examine unprocessed raw data before applying a classification technique such as Artificial Neural Networks (ANN). Artificial Neural Networks (ANN} are being applied in a growing number of applications in many fields because they can satisfactorily resolve complex analytical problems. Although simple in structure, the large number of interconnections in an Artificial Neural Network (ANN) has an interesting potential for calculations. Actually, Artificial neural Networks (ANN) have already been used in medicine with excellent results, even in urology, where the main applications have been related to the diagnosis of cancer and other disorders, although they have never been used for classifying kidney stones.

The main purpose of this study is to evaluate the possibility of using NIR-HSI and Artificial Neural Networks (ANN) for the characterisation and classification of kidney stones, as this could be useful for medical diagnosis, improving the conventional characterisation methods used to date.

The proposed procedure for the characterisation of kidney stones includes the following phases:

- Selecting a variety of types of kidney stones
- Cutting the stones to expose the inner part and obtain a surface that is as flat as possible
- Stone surfaces are analysed by HSI in combination with Factor Analysis (FA), Principal Component Analysis (PCA) and Artificial Neural Networks (ANN).

The hyperspectral measurements obtained using a spectral camera in the NIR region consist of a spectrograph to create the images and a camera. It works as a push-broom type line scan camera that provides full contiguous hyperspectral data for each pixel along the imaged line. The spectral camera is housed on a laboratory platform for inspecting different samples. The platform is fitted with a conveyor belt that enables movement of the particles to obtain the spectra, a frame to support the spectral sensor and the light source, a control console also used as a work table, and a place for a digital computer and a monitor. The device is controlled by a PC equipped with acquisition/preprocessing software.

Spectra of the samples are measured by mounting them on a plastic support with the inner side up, making the nucleus visible to the detector, to minimise any error in the measurements as a result of the vibration of the conveyor belt.

The stones are analysed by selecting a series of regions of interest (ROI) on the surface of the sample. Even though the analysis by Regions of Interest (ROI) loses the information for each individual pixel, it provides two main advantages. In the first place, selecting certain areas instead of individual pixels smoothes out the surface of the sample, as some pixels may be slightly different from the rest of the sample. This difference could be explained by the nature of the sample, organic matter could have been trapped inside the structure of the stone during the crystallisation process. In second place, the amount of information to be processed is much less than for pixel-by-pixel analysis.

Furthermore, Principal Component Analysis (PCA) is performed on each individual pixel of the image, thus revealing the capability of this technique for the classification of entire images.

The raw data is processed using three different methods:

- Factor Analysis (FA)
- Principal Component Analysis (PCA)
- Artificial Neural Networks (ANN)

Although Factor Analysis (FA) and Principal Component Analysis (PCA) share the same main purpose, that is, reducing the number of system variables by finding latent connections between real variables and different mathematical properties, they do have differences that enable obtaining different information.

Factor Analysis (FA) is based on the correlation between real variables, and the number of variables considered as system descriptors is selected in accordance with the associated eigenvalue. Several different criteria have been suggested for deciding how many factors are descriptive of the system, and each case has its own solution, as there are no general rules that fit all cases. Kaiser's rule, which points out that only factors with an associated eigenvalue of more than 1 are representative of system, has been applied in this study. All the other factors represent linear combinations of real variables; therefore, they do not describe any new system component.

On the other hand, Principal Component Analysis (PCA) is based on the variance of the data. Thus, the main key component for the system is the direction where data have highest variance.

Given these properties, Factor Analysis (FA) is used to check how many different components can be distinguished in all samples, whereas Principal Component Analysis (PCA) is used for selecting variables.

Artificial Neural Networks (ANN) are considered a sophisticated and powerful software tool that solves difficult analytical problems by learning from real cases. Despite the different structures that Artificial Neural Networks (ANN) can adopt, the structure used in this study always had three layers: input, hidden and output.

The algorithm selected for the learning process is back propagation. Optimisation of Artificial Neural Networks (ANN) is performed by minimising the RMS error while changing the number of nodes in the hidden layer.

Factor Analysis (FA) (as well as PCA and ANN) is performed using software. The data used for this analysis are the spectra corresponding to different groups of components that represent the full range of substances studied. The weight of the eigenvalue associated with each factor is lower for the least important factors. Factor Analysis (FA) distinguishes the main species comprising the kidney stones. Principal Component Analysis (PCA) is therefore proposed to create a model capable of classifying the different types of kidney stones studied.

Principal Component Analysis (PCA) applies software to each individual group of kidney stones to determine the existence of outliers in the raw data. The elimination of any point from the original samples is a crucial stage as reducing the amount of data also reduces the variability of the system. Furthermore, there is no way of being completely sure whether a sample is correctly considered as an outlier. Thus, the criteria for determining outliers were based on the variability of the sample spectra. The data was cleaned to obtain a more reliable database. Considering the biological nature of the samples, the structure of crystals in the kidney stone may contain organic material that can be maintained in the structure of the stone during the growth process. Furthermore, given the nature of the samples, the measured surface may not be completely regular, thus creating a reflectance value which differs from the group mean.

Each Region of Interest (ROI) is considered a different sample in terms of software. It takes into account the full range of variables for calculations, thus being able to check whether any of the regions of a sample, or a complete sample, is really different from the rest.

They represent the principal components (PC) system, making possible the detection of outliers in the raw data.

After having cleaned the data, a Principal Component Analysis (PCA) was performed on the different main groups of kidney stones to create a model that can correctly classify the kidney stones studied. Effectiveness is verified by cross-validation, and all data are focused on the mean.

In order to obtain a wider distribution of the data variance, a first derivative was performed on the raw data. This stage required previous smoothing of the data. The results to predict samples are better than non-derived data; therefore, the first derivative increases the slight differences between groups of kidney stones.

When working with Principal Component Analysis (PCA), it was observed that the best classification results are obtained if the mean  reflectance spectra are smoothed before calculating the first derivative.

The Artificial Neural Networks (ANN) model is applied to reduce the number of variables in the system. The use of these Artificial Neural Networks (ANN) for processing hyperspectral data shows results similar to those obtained with conventional techniques. However, it is important to note that conventional methods require trained operators while Artificial Neural Network (ANN) classification is performed irrespective of operator skills. Furthermore, the diagnosis is faster, increasing the patient's quality of life.

**Description of the drawings**

In addition to the description provided here and in order to aid in a better understanding of the characteristics of the invention, and in accordance with a preferred example of a practical embodiment, an integral part of the description is a set of drawings where, in an illustrative and not limiting sense, the following is shown:

Figure 1 shows the eigenvalues for the seven main groups of kidney stones in the experimental study.

Figure 2 shows the PC1 vs. PC2 scores for 27 samples of anhydrous uric acid.

Figure 3 shows the variance of the model for each Principal Component (PC).

Figure 4 shows the loads of each variable for each of the first 7 Principal Components in the experimental part.

Figure 5 shows the PC1 vs. PC2 scores for the 7 main groups of kidney stones in the experimental part.

Figure 6 shows the 3D representation of PC1, PC2 and PC3 scores in the experimental part.

Figure 7 shows the PC1 vs. PC3 scores.

Figure 8 shows one example for optimisation of Artificial Neural Network (ANN)

**Preferred embodiment of the invention**

The proposed invention is a novel method for characterization and classification of kidney stones, which uses the Hyperspectral Imaging technique, combined with Artificial Neural networks (ANN). It consists of the following general steps:

(a) Taking a series of kidney stone samples and cutting them to observe their interior, obtaining the flattest possible surface,
(b) Hyperspectral Imaging (HSI) is applied to obtain the spectra of previously cut kidney stones, selecting a series of Regions of Interest (ROI) and analysing the image using Principal Component Analysis (PCA).
(c) The main species are identified using Factor Analysis (FA).
(d) Outliers are identified using Principal Component Analysis (PCA).
(e) The different types of kidney stones are tested using Principal Component Analysis (PCA).

(f) The data obtained from the Principal Component Analysis (PCA) are subjected to Artificial Neural Networks (ANN) for classification.

Preferred embodiment example

215 samples were selected from a library of more than 1,400 kidney stones for this study. Eleven types of kidney stone components were considered, including mixtures. All the samples were collected in the Urology Department of the Hospital Universitari de Bellvitge, Barcelona (Spain). The selection criteria were based on having as much variability as possible for any type of kidney stone.

The samples were divided into two different groups, depending on their composition. In the first place, the seven main types of kidney stones were considered, as they represent the different compounds usually found in kidney stones: AUA (Anhydrous uric acid), BRU (Brushite), COD (Calcium Oxalate Dihydrate), COM (Calcium Oxalate Monohydrate), CYS (Cysteine), HAP (Hydroxyapatite) and STR (Struvite). In second place, a study was made of four more groups, including mixtures of the first, specifically: UAD (uric acid dihydrate), MXL (mixed Calcium Oxalate and Hydroxyapatite, in layers), MXD (mixed Calcium Oxalate and Hydroxyapatite) and TRA (Dehydrated Calcium Oxalate transformed into Calcium Oxalate Monohydrate). A total of 11 different types of kidney stones were tested.

All samples were sliced with a scalpel to reveal the interior of the stone and, at the same time, to obtain a surface as flat as possible, before analysis via HSI.

Samples were first analysed by stereoscopic microscopy to compare these results with those obtained using the proposed method. This technique is widely used for the characterisation of kidney stones.

A scanning electron microscope (SEM) was used for samples that were not well characterised using this method. In these cases, a small part of the sample was coated with a layer of graphite (the Au peak could overlap the P in the EDS analysis) and processed using an electron microscope. Two different SEM units were used: JEOL JSM-6300 scanning electron microscope (Japan) coupled to an Oxford Instruments Link ISIS-200 Energy Dispersive X-ray Spectrometer (United Kingdom) (Universitat Autònoma de Barcelona -- Autonomous University of Barcelona laboratory) and a HITACHI S2500 scanning electron microscope (Japan) coupled to a Kevex 8000 Energy Dispersive X-ray Spectrometer (United States) (Università di Roma laboratory). The structure of the samples was analysed and EDS analysis performed on some parts of the stone to confirm the basic composition of the sample.

Measurements using Hyperspectral Imaging were made using a NIR spectral camera consisting of an ImSpector N17E imaging spectrograph for the 1000-1700 nm wavelength regions and a Ingaas thermostabilised camera (Specim, Finland). It operates as a push-broom type line scan camera that provides full contiguous hyperspectral data for each pixel along the imaged line. The spectral camera is housed on a laboratory platform developed by DV SRL (Italy) for the inspection of different samples. The platform is fitted with a conveyor belt that enables movement of the particles to obtain the spectra, with a frame to support the spectral sensor and the light source, with a control console also used as a work table and a place for a digital computer and a monitor. The device is fully controlled by a PC with Spectral Scanner™ v. 2.3 acquisition/preprocessing software.

The resolution of the spectra was 7 nm; therefore measurements were measured. The spectrometer was fitted with a 50 mm lens. The image width resolution was 320 pixels, whereas the number of squares, that is, the resolution of the image on the Y axes varied between 200 and 350, depending on the number of samples measured at the time.

The spectra of the samples was measured by mounting them on a plastic support with inner side up, so that the nucleus was visible to the detector to minimise any error in the measurements as a result of vibration of the conveyor belt.

The spectral measurements were calculated as relative reflectance (R), using the following equation:

$$R = \frac{r_s - r_b}{r_w - r_b}$$

where $r_s$ is the measured reflectance, $r_b$ is the measured reflectance for black (baseline interference) and $r_w$ the reflectance for a white standard (100% reflectance).

The stones were analysed by selecting Regions of Interest (ROI) on the sample surface. Five Regions of Interest (ROI) were selected randomly for each sample to provide representative data of the entire surface. Even though the analysis by Regions of Interest (ROI) loses the information for each individual pixel, it has two main advantages. In the first place, selecting certain areas instead of individual pixels smoothes out the surface of the sample, as some pixels may be slightly different from the rest of the sample. This difference could be explained by the nature of the sample, as organic matter could have been trapped inside the structure of the stone during the crystallisation process. In second place, the amount of information to be processed is much less than for pixel-by-pixel analysis.

Furthermore, Principal Component Analysis (PCA) is performed on each individual pixel of the image, thus revealing the capability of this technique for the classification of entire images.

By stages, three different methods were used to analyse the data: Factor Analysis (FA), Principal Component Analysis (PCA) and Artificial Neural Networks (ANN).

Although Factor Analysis (FA) and Principal Component Analysis (PCA) share the same main objective, that is, reduce the number of system variables by finding latent connections between real variables and different mathematical properties, they do have differences that enable obtaining different information.

Factor Analysis (FA) is based on the correlation between real variables, and the number of variables considered system descriptors is selected in accordance with the associated eigenvalue. Several different criteria have been suggested for deciding how many factors are descriptive of the system, and each case has its own solution, as there are no general rules that fit all cases. Kaiser's rule, indicating that only factors with an associated eigenvalue of more than 1 are representative of system has been applied in this study. All the other factors represent linear combinations of real variables; therefore, they do not describe any new system component.

On the other hand, Principal Component Analysis (PCA) is based on the variance of the data. Thus, the main key component for the system is the direction where the data have greatest variance.

Because of these properties, Factor Analysis (FA) is used to check how many different components can be distinguished in all samples, whereas Principal Component Analysis (PCA) is used for the selection of variables.

Artificial Neural Networks (ANN) are considered a sophisticated and powerful software tool that solves difficult analytical problems by learning from real cases. Despite the many different structures that Artificial Neural Networks (ANN) can adopt, the structure used in this study always had three layers: input, hidden and output.

The algorithm selected for the learning process is back propagation. Optimisation of Artificial Neural Networks (ANN) is performed by minimising the RMS error while changing the number of nodes in the hidden layer.

The Hyperspectral Imaging data was processed using the following software: for FA, STATISTICA, Tulsa, OK, United States; for Principal Component Analysis (PCA), The Unscrambler v 9.1, Camo Process, Oslo, Norway; for image processing, MATLAB v 7.0, MA, United States (PLS Toolbox by Eigenvector Research, Inc.) and TRAJAN v 3.0, Horncastle, United Kingdom for ANN.

STATISTICA software was used for Factor Analysis (FA) of the data. The data used for this analysis are the spectra corresponding to the seven groups of components representing the full variety of substances studied.

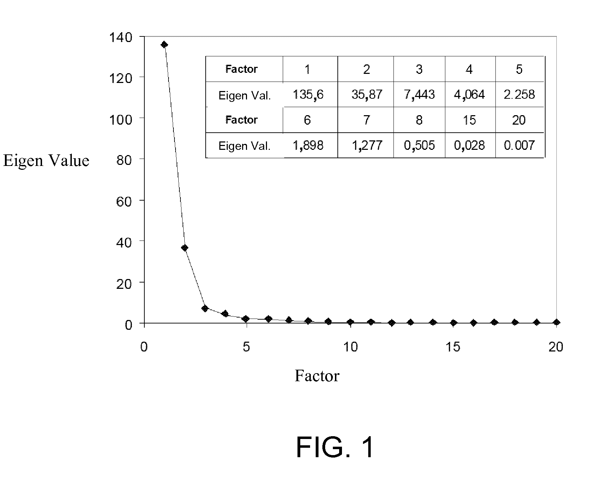

Figure 1 shows how the weight of the eigenvalue associated with each factor is lower for each less important factor, the seventh factor being the last to have a value of more than 1. According to Kaiser's rule, this could be the last factor describing the system. As a result, it can be seen how Factor Analysis (FA) distinguishes between the main species forming the kidney stones. Considering these results, Principal Component Analysis (PCA) is proposed to create a model capable of classifying the different types of kidney stones studied.

In the first place, Principal Component Analysis (PCA) was performed on each individual group of kidney stones, each with a different chemical composition, to determine the existence of outliers in the raw data. These sample groups included the seven main groups of kidney stones.

The elimination of any point from the original samples is a crucial stage as reducing the amount of data also reduces the variability of the system. Furthermore, there is no way of being completely sure whether a sample is correctly considered as an outlier. Therefore, the criteria for determining outlier values were based on the variability of the spectra of the samples. The data was cleaned to obtain a more reliable database. Considering the biological nature of the samples, the structure of crystals in the kidney stone may contain organic material that can be maintained in the structure of the stone during the growth process. Furthermore, given the nature of the samples, the measured surface may not be completely regular, thus creating a reflectance value which differs from the group mean.

Analysis of the samples of anhydrous uric acid (AUA) will be shown as an example, considering that all other of groups were analysed in the same way.

The Unscrambler software was used to perform Principal Component Analysis (PCA) on 5 Regions of Interest (ROI) for each sample of anhydrous uric acid (AUA). Therefore, each Region of Interest (ROI) was considered a different sample in terms of software. In this first stage, we took into account the full range of variables for the calculations, that is, 97 variables. Organizing the data in this way enables checking whether any of the regions of a sample, or a complete sample, are really different from the others.

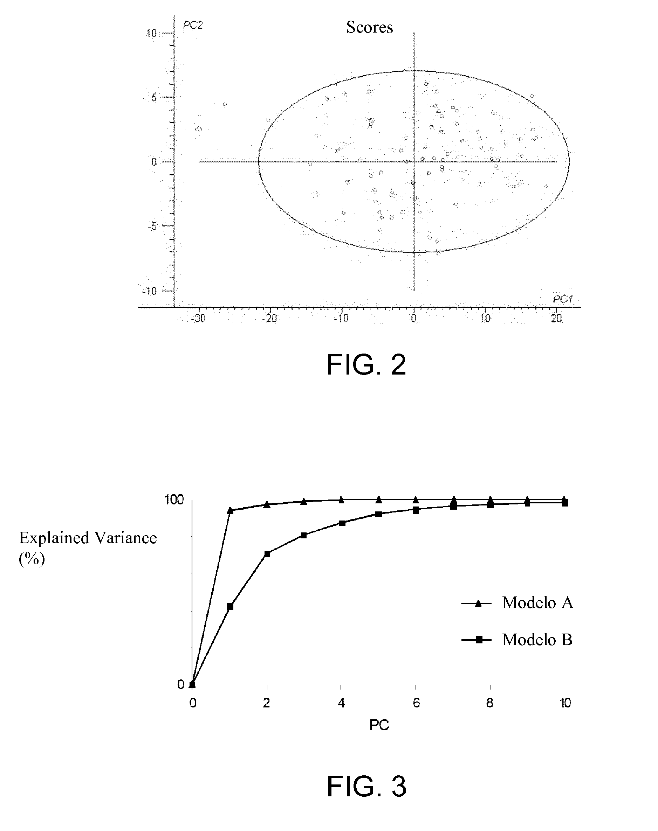

Representing the principal components (PC) of the system enables the detection of outliers in the raw data. The distribution of the values of the PC1 and PC2 scores for all the Regions of Interest (ROI) of anhydrous uric acid (AUA) is shown in figure 2. This representation includes Hotelling's $T^2$ ellipse showing the distance of every point from the centre of the model. In our case, this ellipse represents 95% of the model variance.

Careful analysis of figure 2 clearly reveals that four Regions of Interest (ROI) are outside Hotelling's ellipse for negative PC1 values. As all these Regions of Interest (ROI) correspond to the same sample, it is considered to be an outlier.

On the other hand, there are two Regions of Interest (ROI) outside the ellipse with positive PC1 values representing different samples. However, these Regions of Interest (ROI) are not deleted from the complete collection of data so as not to overadjust the system.

After applying this procedure to all the different groups of kidney stones, it is obvious that only one sample of anhydrous uric acid (AUA) and another of calcium oxalate monohydrate (COM) are probable outliers.

After having cleaned the data, a Principal Component Analysis (PCA) was performed on the seven groups of kidney stones to create a model capable of correctly classifying the kidney stones studied.

This model was created using all 97 variables for the calculation. The effectiveness was verified by crossed validation and all the data were centred on the mean.

In the first place, the model was calculated directly on the basis of the acquired reflectance data. Figure 3 Model A shows that, in this case, the first two principal components (PC) represent 95% of the variance of the model, the rest of the principal components (PC) therefore having a relatively low value.

In order to obtain a wider distribution of the data variance, a first derivative was performed on the raw data. This stage required previous smoothing of the data which was performed using the Savitzky-Golay algorithm and a 5-point window. In this case, there is a widely distributed explanation of the variance; more specifically, up to 7 principal components (PC) are required to explain 96% of the variance for the model (Figure 3 Model B).

Nevertheless, as can be observed in the following section, the results for predicting samples are better than those from non-derived data. Therefore, it can be concluded that the first derivative increases the slight differences between the seven groups of kidney stones. For this reason, the first derivative of the spectra was used for additional processing of the data.

Multivariable methods can give a much greater amount of information than univariable ones, although many interval variables measured may not provide valuable data, only interference. In this regard, it is possible to take advantage of the fact that one of the main purposes of Principal Component Analysis (PCA) is to reduce the number of variables, taking only the information contained in real variables. Actually, not all regions of the NIR spectrum provide information relevant to the model. This means that selecting the wavelengths with the greatest classification capabilities reduces the interference in the system, increasing its precision and simplifying the calculations for the model.

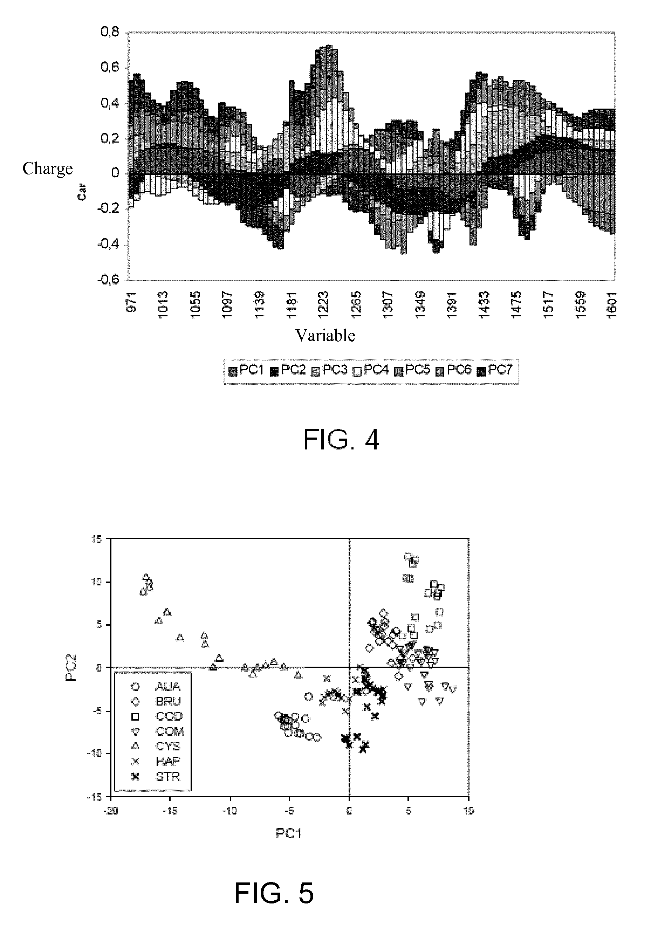

The wavelengths to be used for the calculations may be the ones with the highest load value for each Principal Component (PC). The representation of the loads for each wavelength and each Principal Component (PC) (Figure 4) is a clear way of visualising which wavelengths are the most important.

Based on the information in Figure 4 and the representation of the values of the scores for the two principal components (PC), it is also possible to define the variables describing each compound.

The representation of PC1 vs. PC2 scores (Figure 5) is provided as an example. It shows that both Anhydrous Uric Acid (AUA) and Cysteine (CYS) have negative PC1 values but the opposite sign for PC2 and this means that PC2 is clearly differentiating between these two components.

Figure 5 shows that PC2 is well defined with positive load values for the wavelengths of the 1188-1230 nm and 1440-1542 nm intervals. All these energies are associated with vibrations of CH bonds. This association is in perfect agreement with the structure of Cysteine, as this is the only component of the series studied that has C-H bonds. On the other hand, the 971-978 nm and 1083-1167 nm wavelengths have negative load values for PC2. In this case, the vibrations are associated with Ar-OH bonds, which can only be related to anhydrous uric acid (AUA), by tautomeric equilibrium with Cysteine structure.

In addition to the data for Cysteine (CYS) and Anhydrous Uric Acid (AUA), other information can be obtained to define the rest of compounds in Table 1, as shown below. Obviously, the vibration differentiating Calcium Oxalate Monohydrate (COM) and Calcium Oxalate Dihydrate (COD) is the band for water.

Table 1 below shows the main information extracted from the scores and loads for each group of kidney stones:

| Type of kidney stone | Characteristic PC | λ with greater *load* (nm) | Associated NIR vibrations |
|---|---|---|---|
| CYS | PC1<0 PC2>0 | 1188-1230, 1440-1542 | CH, $CH_2$, $CH_3$ |
| AUA | PC1<0 PC2>0 | 971-978, 1083-1167 | Ar-OH |
| COD | PC3>0 PC2<0 | 1426-1475 | $H_2O$ |
| COM | PC3<0 PC4>0 | | |
| BRU | PC3<0 PC4<0 | 1223-1244 | CH |
| HAP | | Not well defined | |
| STR | | | |

As can be seen, the NIR bands defining Brushite (BRU) are in the interval where C-H vibrations appear. This could be due to the especially large amount of organic material contained in Brushite (BRU) calculi as there are no C-H bonds in the structure of brushite.

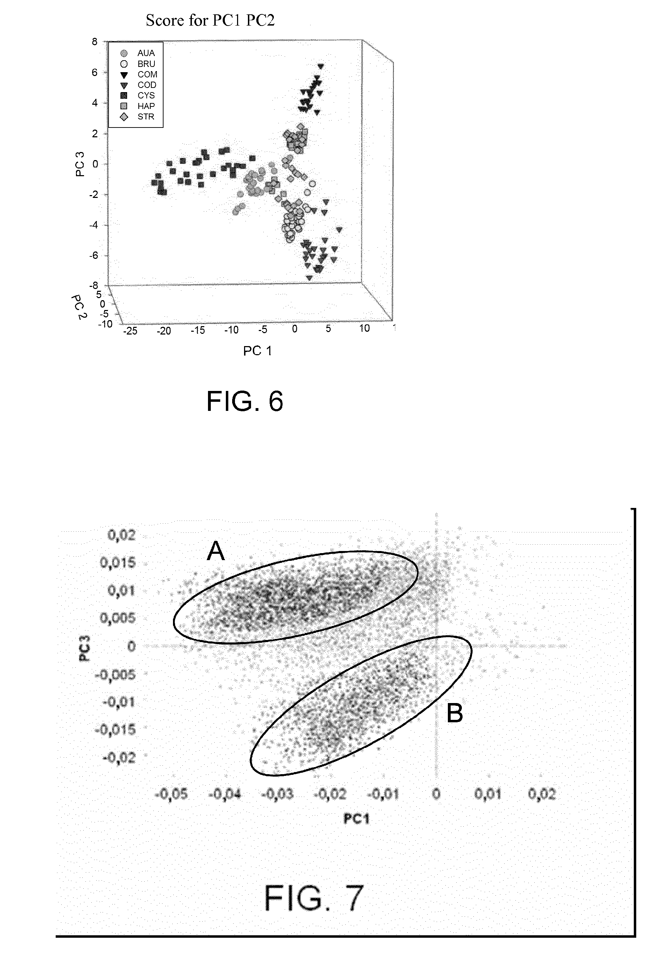

The main drawback of Principal Component Analysis (PCA) is that it is incapable of separating Hydroxyapatite (HAP) and Struvite (STR), as can be clearly seen in figure 6, where these two compounds appear as two overlapping groups. This is because the composition of both compounds is very similar. Struvite calculi are basically Hydroxyapatite stones with varying amounts of Struvite crystals scattered around inside the stone. This means that a large part of the sample is similar to one pure Hydroxyapatite (HAP) kidney stone.

In spite of the unique exception of Hydroxyapatite (HAP) and Struvite (STR) kidney stones, Principal Component Analysis (PCA) has proved to be useful for the classification of the main components of kidney stones, as shown in figure 6.

Different Principal Component Analyses (PCA) were also performed on the seven groups of kidney stones, this time directly using the hyperspectral cube data and MATLAB software.

One sample of each type of kidney stone was analysed to obtain a pixel-by-pixel classification. Just like for Principal Component Analysis (PCA), the data used were the first derivative of the spectra and centred on the mean.

Some samples considered pure compounds (as classified by Stereoscopic and Scanning Electron Microscopy (SEM) were used to create a model for the later classification of unknown samples. The classification criteria were determined by selecting which group each compound corresponded to on a plot of the scores.

The different compounds were identified by interpreting the colours obtained when creating RGB images reconstructed from the hyperspectral cube. When using this type of representation, the three Principal Components (PC) used for the image determine the colour of each type of kidney stone.

The different colours for each type of kidney stone mean that each one can be distinguished from the others, except for Struvite and Hydroxyapatite. In fact, the results obtained when analysing the hyperspectral cube coincide perfectly with those obtained by taking Regions of Interest (ROI) of the samples. The method and conditions used for analysis are similar in both cases.

Furthermore, the software enables selecting one group of pixels from the representation of the scores. Thus, it is possible to identify which area of a sample or group of samples contains a given compound once the values of the scores for each of them are known.

Figure 7 shows the values of the scores for PC1 and PC3 in a sample that mainly contains Calcium Oxalate Monohydrate (COM) and Calcium Oxalate Dihydrate (COD). Among the agglomeration of pixels shown in figure 7 it is possible to identify differentiated zones for Calcium Oxalate Monohydrate (COM) and Calcium Oxalate Dihydrate (COD), as well as an intermediate zone of pixels.

When working with Principal Component Analysis (PCA), it was observed that the best classification results are obtained if the mean reflectance spectra are smoothed before calculating the first derivative. Therefore, these data were processed using TRAJAN software to create the Artificial Neural Network (ANN) model.

The raw data of the spectra consist of up to 97 wavelengths and a selection was made of 50 of them to reduce the number of variables in the system. The selection criteria were based on the load values for each wavelength according to the data in Figure 4 to create a matrix of 140 samples (7 primary types of kidney stones) and 50 variables for analysis.

The Artificial Neural Network (ANN) to be optimised was defined as: inputs-number of nodes in the hidden layer-outputs, where the inputs represent the number of samples to create the model and the outputs the number of types of kidney stones, that is (140, n, 7). The output value is a single variable that can have 7 different values.

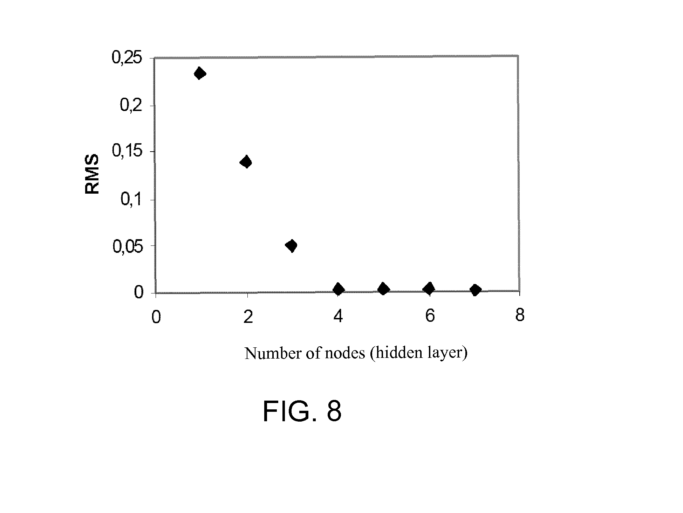

After optimising the Artificial Neural Network (ANN), it was observed (Figure 8) that the ideal number of nodes in the hidden layer was 4 because, after this point, no further reduction of the RMS error was detected.

The model was applied to the seven groups of main compounds of kidney stones using these conditions. The results, checked by cross validation leaving one value out, showed an accuracy of up to 100% for the classification.

Bearing these promising results in mind, the following stage was to further reduce the number of variables in the system so that the predicted advantages of reducing the variables became more obvious. In this case, even though 30 wavelengths were selected, the accuracy of the classification continued to be 100%.

When taking even fewer variables for the calculations, the percentage of correct classification fell to 95% at the most. Therefore, the final election was the model using 30 wavelengths.

However, due to the importance of an exact classification for correct diagnosis of the patient, the really interesting objective is the classification of 11 groups of different types of kidney stones, that is, the seven main compounds and their mixtures.

Therefore, it was necessary to optimise a new Artificial Neural Network (ANN) to create a different model capable of classifying these, much more complex data. With this in mind, 50 wavelengths were entered as variables with the structure of the ANN being (215, 13, 11). The number of nodes in the hidden layer was verified using the same procedure as for the previous model.

In this case, the number of samples correctly classified was 93%. Even though the correct classification rate for the model with 11 compounds was lower than that of 7, it should be noted that the complexity of the samples entered into the new model represents slightly different results. In any case, these results may be considered as acceptable, considering the simplicity of the measurements required.

This shows the suitability of Hyperspectral Imaging (HSI) combined with the use of Artificial Neural Networks (ANN) for the characterisation of kidney stones. The regions of the NIR reflectance spectra that best enable the correct classification of the different components have been identified.

**Claims**

1. Method for the characterisation and classification of kidney stones, **characterised in that** it comprises the following stages:

   (a) Taking a series of kidney stone samples and cutting them to observe their interior, obtaining the flattest possible surface,
   (b) The technique of Hyperspectral Imaging (HSI) is applied to obtain the spectra of previously cut kidney stones, selecting a series of Regions of Interest (ROI) and analysing the image using Principal Component Analysis (PCA)
   (c) The main species are identified using Factor Analysis (FA)
   (d) Outliers are identified using Principal Component Analysis (PCA)
   (e) The different types of kidney stones are analysed using Principal Component Analysis (PCA)
   (f) The data obtained from the Principal Component Analysis (PCA) are subjected to Artificial Neural Networks (ANN) for classification.

2. Method for the characterisation and classification of kidney stones, according to claim 1, **characterised in that** the spectra are obtained using the technique of Hyperspectral Imaging (HSI), where a camera combined with an imaging spectrograph captures the spectrum for each pixel of the acquired image of the sample, mounting the kidney stone samples on a support with inner side facing upwards to view the nucleus through a detector.

| Factor | 1 | 2 | 3 | 4 | 5 |
|---|---|---|---|---|---|
| Eigen Val. | 135,6 | 35,87 | 7,443 | 4,064 | 2.258 |
| Factor | 6 | 7 | 8 | 15 | 20 |
| Eigen Val. | 1,898 | 1,277 | 0,505 | 0,028 | 0.007 |

FIG. 1

FIG. 2

FIG. 3

FIG. 4

FIG. 5

Score for PC1 PC2

FIG. 6

FIG. 7

FIG. 8

# INTERNATIONAL SEARCH REPORT

| | International application No. |
|---|---|
| | PCT/ES2012/070239 |

A. CLASSIFICATION OF SUBJECT MATTER

**See extra sheet**

According to International Patent Classification (IPC) or to both national classification and IPC

B. FIELDS SEARCHED

Minimum documentation searched (classification system followed by classification symbols)
G01N, G06T, G01J, G06F, A61B

Documentation searched other than minimum documentation to the extent that such documents are included in the fields searched

Electronic data base consulted during the international search (name of data base and, where practicable, search terms used)

INVENES, EPODOC, WPI, INVENES, TXTE, TXTF, NPL, MEDLINE BIOSIS, EMBASE

C. DOCUMENTS CONSIDERED TO BE RELEVANT

| Category* | Citation of document, with indication, where appropriate, of the relevant passages | Relevant to claim No. |
|---|---|---|
| A | 2005, BOSKEY A. L., MENDELSOHN R. "Infrared spectroscopic characterization of mineralized tissues." Vib. Spectrosc. (2005) Vol. 38, pages 107-114. The whole document. | 1-2 |
| A | 2008, HAMED AKBARI et al. " Wavelet-based compression and segmentation of hyperspectral images in surgery." Lecture Notes in Computer Science (2008) Vol. 5128/2008, pages 142-149. The whole document. | 1-2 |
| A | WO 2010/019515 A2 (BOARD OF REGENTS, THE UNIVERSITY OF TEXAS SYSTEM) 18.02.2010. The whole document. | 1-2 |

☒ Further documents are listed in the continuation of Box C.     ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | "&" document member of the same patent family |

| Date of the actual completion of the international search | Date of mailing of the international search report |
|---|---|
| 31/07/2012 | **(03/08/2012)** |
| Name and mailing address of the ISA/ <br><br> OFICINA ESPAÑOLA DE PATENTES Y MARCAS <br> Paseo de la Castellana, 75 - 28071 Madrid (España) <br> Facsimile No.: 91 349 53 04 | Authorized officer <br> M. García Bueno <br><br><br> Telephone No. 91 3497000 |

Form PCT/ISA/210 (second sheet) (July 2009)

## INTERNATIONAL SEARCH REPORT

| | International application No. |
|---|---|
| | PCT/ES2012/070239 |

| C (continuation). | DOCUMENTS CONSIDERED TO BE RELEVANT | |
|---|---|---|
| Category * | Citation of documents, with indication, where appropriate, of the relevant passages | Relevant to claim No. |
| A | 2003, HAMID A., DWIVEDI U.S., SINGH T.N., GOPI KISHORE M., MAHMOOD M., SINGH H., TANDON V., and SINGH P.B. "Artificial neural networks in predicting optimum renal stone fragmentation by extracorporeal shock wave lithotripsy: a preliminary study." British Journal Urology International (2003) Vol. 91, pages 821-824. The whole document. | 1-2 |
| A | 2010, GOYAL N. K., KUMAR A., TRIVEDI S., DWIVEDI U. S., SINGH T. N., SINGH P. B. "A comparative study of artificial neural network analysis and multivariate regression analysis to analyze optimum renal stone fragmentation by extracorporeal shock wave lithotripsy." Saudi Journal of Kidney Diseases and Transplantation (2010) Vol. 21, pages 1073-1080. The whole document. | 1-2 |

Form PCT/ISA/210 (continuation of second sheet) (July 2009)

## INTERNATIONAL SEARCH REPORT

Information on patent family members

| International application No. |
| --- |
| PCT/ES2012/070239 |

| Patent document cited in the search report | Publication date | Patent family member(s) | Publication date |
| --- | --- | --- | --- |
| WO2010019515 A | 18.02.2010 | US2010056928 A | 04.03.2010 |

**INTERNATIONAL SEARCH REPORT**

| International application No. |
| --- |
| PCT/ES2012/070239 |

**CLASSIFICATION OF SUBJECT MATTER**

*G01N21/00* (2006.01)
*G06T1/40* (2006.01)
*G01J3/40* (2006.01)
*G06F19/00* (2011.01)
*A61B6/00* (2006.01)

Form PCT/ISA/210 (extra sheet) (July 2009)